(19) Europäisches Patentamt
European Patent Office
Office européen des brevets

(11) Veröffentlichungsnummer: **0 497 239 A1**

# (12) EUROPÄISCHE PATENTANMELDUNG

(21) Anmeldenummer: **92101258.9**

(51) Int. Cl.5: **C07C 255/50**

(22) Anmeldetag: **27.01.92**

(30) Priorität: **30.01.91 DE 4102671**

(43) Veröffentlichungstag der Anmeldung:
**05.08.92 Patentblatt 92/32**

(84) Benannte Vertragsstaaten:
**DE ES FR GB IT**

(71) Anmelder: **HOECHST AKTIENGESELLSCHAFT**
**Postfach 80 03 20**
**W-6230 Frankfurt am Main 80(DE)**

(72) Erfinder: **Pfirmann, Ralf, Dr.**
**Karlstrasse 1c**
**W-6103 Griesheim(DE)**
Erfinder: **Papenfuhs, Theodor, Dr.**
**Heinrich-Bleicher-Strasse 40**
**W-6000 Frankfurt am Main 50(DE)**

## (54) 2-Chlor-4,5-difluorbenzonitril und Verfahren zu seiner Herstellung.

(57) 2-Chlor-4,5-difluorbenzonitril und Verfahren zu seiner Herstellung, indem man 2,4-Dichlor-5-fluorbenzonitril mit Kalium-, Rubidium- oder Cäsiumfluorid oder einem Gemisch daraus in einem dipolar aprotischen Lösungsmittel, gegebenenfalls in Gegenwart eines Phasentransferkatalysators, bei Temperaturen von etwa 80 bis 240 °C, umsetzt.

EP 0 497 239 A1

Die vorliegende Erfindung betrifft 2-Chlor-4,5-difluorbenzonitril und ein Verfahren zu seiner Herstellung. Die neue Verbindung stellt ein wertvolles Zwischenprodukt zur Herstellung antibakterieller Mittel dar.

Zentrale Vorprodukte für die Synthese von hochwirksamen, antibakteriellen Mitteln der Chinoloncarbonsäurereihe sind 2,4-Dihalogen-5-fluorbenzoesäuren, die nach literaturbekannten Methoden (2,4,5-Trifluorbenzoesäure: J. P. Sanchez, J. M. Domagala, S. E. Hagen, C. L. Heifetz, M. P. Hutt, J. B. Nichols, A. K. Trehan, J. Med. Chem. 31,983-991 (1988); Abbott Lab., EP 227 088, 1.7.87; Bayer AG, DE 3 600 891, 16.7.87; DE 3 420 743, 15.12.85; Daiichi Seiyaku Co. Ltd., JP 60 072 885, 24.4.85; EP 191 185, 20.8.86; 2,4-Dichlor-5-fluorbenzoesäure: Bayer AG, DE 3 702 393, 11.8.88; DE 3 615 767, 12.11.87; DE 36 01 567 (23.7.87); DE 3 600 891 (16.7.87); DE 3 522 406 (2.1.87); DE 3 517 535 (20.11.86); DE 3 641 312 (9.6.88); 2-Chlor-4,5-difluorbenzoesäure: Pfizer Inc., EP 342 849, 23.11.89; EP 321 191, 21.6.89; Asahi Glass Co. Ltd., EP 303 291, 15.2.89) zu den Wirkstoffen umgesetzt werden können.

Gegenstand der Erfindung sind das 2-Chlor-4-5-difluorbenzonitril und Verfahren zu seiner Herstellung, indem man 2,4-Dichlor-5-fluorbenzonitril mit Kalium-, Rubidium- oder Cäsiumfluorid oder einem Gemisch aus diesen Fluoriden in einem dipolar aprotischen Lösungsmittel, gegebenenfalls in Gegenwart eines Phasentransferkatalysators, bei Temperaturen von etwa 80 bis etwa 240 °C, vorzugsweise 140 ° bis etwa 190 °C, umsetzt.

Mischungen aus Kalium- und Cäsiumfluorid sind bevorzugt.

Als dipolar aprotische Lösungsmittel kommen beispielsweise Tetramethylensulfon, Dimethylsulfoxid, Tetramethylensulfoxid, Dimethylacetamid, Dimethylformamid oder N-Methylpyrrolidon in Betracht.

Geeignete Phasentransferkatalysatoren sind Ammonium- oder Phosphoniumsalze, wie beispielsweise Tetra-($C_1$-$C_{18}$-alkyl)-ammoniumchloride oder -bromide, wie Tetramethylammoniumchlorid oder -bromid oder Octadecyltrimethylammoniumchlorid oder -bromid, ferner Tetra-($C_1$-$C_{18}$-alkyl)-phosphoniumchloride oder -bromide, Tetraphenylphosphoniumchlorid oder -bromid, [$(Phenyl)_m$-($C_1$-$C_{18}$-$alkyl)_n$]-phosphoniumchloride oder -bromide, worin m = 1 bis 3 und n = 3 bis 1 und die Summe m + n = 4 ist.

Es war überraschend, daß sich die Halogenaustauschreaktion nach dem Austausch eines Chloratoms so verlangsamt, daß sich mit beträchtlich hoher Selektivität und ohne größeren zweifachen Chloraustausch das 2-Chlor-4,5-difluorbenzonitril herstellen und isolieren läßt.

Ebenfalls überraschend war die hohe Selektivität, mit der das Chloratom in der 4-Position ausgetauscht wird. Die isomere Verbindung kann nur zu Beginn der Reaktion nachgewiesen werden. Die Isomereneinheit des destillierten Produktes beträgt über 98 %.

Die Aufarbeitung erfolgt durch Rektifikation. Hierbei wird neben dem 2-Chlor-4,5-difluorbenzonitril, das in 50 bis 55 %iger Ausbeute isoliert werden kann, 2,4,5-Trifluorbenzonitril in 18 bis 20 %iger Ausbeute erhalten. Das letztgenannte Nitril kann nach Hydrolyse zur 2,4,5-Trifluorbenzoesäure ebenfalls zur Synthese von antibakteriellen Mitteln verwendet werden.

Die nach dem erfindungsgemäßen Verfahren erhältlichen Benzonitrile bzw. die aus ihnen in sehr guten Ausbeuten zu erhaltenden entsprechenden Benzoesäuren besitzen gegenüber den 2,4-Dichlor-5-fluorverbindungen den Vorteil, daß bei der Synthese antibakterieller Mittel höhere Ausbeuten erhalten werden.

Formelschema der Umsetzung:

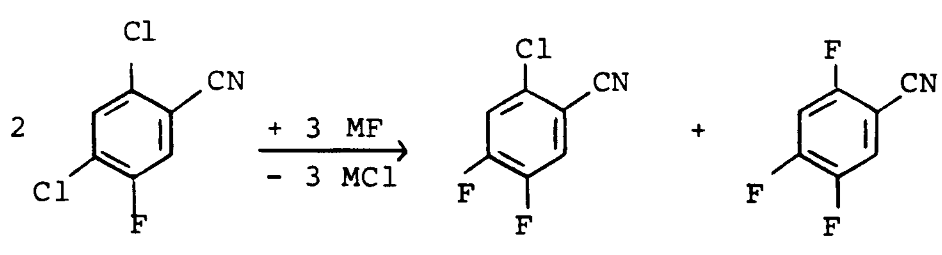


(M = K, Rb, Cs)

Das erfindungsgemäße Verfahren kann sowohl bei Atmosphärendruck als auch bei Unter- oder Überdruck durchgeführt werden.

Das nachstehende Beispiel erläutert das Verfahren, ohne es darauf zu beschränken.

Beispiel

In 350 g Tetramethylensulfon (Sulfolan) werden 136.8 g (2.2 mol) einer Mischung aus Kaliumfluorid und Cäsiumfluorid (9:1) eingetragen. Dazu gibt man noch 5.1 g Octadecyltrimethylammoniumchlorid und destilliert im Vakuum (3 Torr/400 Pa) 50 g des Lösungsmittels ab. Bei 180 °C gibt man schließlich 190 g (1 mol) 2,4-Dichlor-5-fluorbenzonitril hinzu und rührt 2 h kräftig bei dieser Temperatur. Nach dieser Zeit läßt sich GC-analytisch keine Ausgangsverbindung mehr nachweisen. Man entfernt die Reaktionssalze durch Filtration, wäscht mit frischem Sulfolan (je zweimal 50 ml) nach und destilliert bei 28 Torr (3,7 kPa). Es gehen 28.7 g (0.183 mol/18 %) 2,4,5-Trifluorbenzonitril bei 68 °C über, gefolgt von 92.7 g (0.534 mol/53 %) 2-Chlor-4,5-difluorbenzonitril (Sdp. 107 °C). Man erhält jeweils Zwischenfraktionen, die in Folgeansatz bzw. Folgedestillation zurückgeführt werden. Die Struktur wird durch die nachstehenden spektroskopischen Daten belegt; die NMR-Spektren konnten durch Spektrensimulation reproduziert werden.

Ep.34.0 °C

$^1$H-NMR ($CDCl_3$/Tetramethylsilan):
$\delta$ = 7.39 (ddd, 1H, J = 0.34 Hz, J = 9.43 Hz, J = 6.87 Hz, Ar-$H^3$)
7.53 (ddd, 1H, J = 0.34 Hz, J = 7.78 Hz, J = 9.15 Hz, Ar-$\underline{H}^6$)
$^{19}$F-NMR ($CDCl_3$/$CF_3CO_2H$):
$\delta$ = -124.35 (ddd, 1F, J = 7.78 Hz, J = 9.43 Hz, J = 21.13 Hz, Ar-$F^3$)
-135.77 (ddd, 1F, J = 6.87 Hz, J = 9.15 Hz, J = 21.13 Hz, Ar-$\underline{F}^5$)
IR [$cm^{-1}$]: $\tilde{\nu}$ = 3120, 3060, 2235, 1795(w), 1760(w), 1725(w), 1610, 1590, 1570, 1495, 1390, 1305, 1195, 1185, 1140, 1010, 900, 865, 810, 640, 620, 490

| $C_7H_2ClF_2N$ (173.55) | | | | | |
|---|---|---|---|---|---|
| ber.<br>gef. | C 48.45<br>C 48.30 | H 1.16<br>H 1.10 | N 8.07<br>N 8.20 | F 21.89<br>F 22.20 | Cl 20.43<br>Cl 20.30 |

MS (EI/70 eV): $\underline{m/z}$ (%) = 61(4.5), 75(4.8), 88(17.2), 112(5.5), 118(2.7), 138(18.3), 173(100), 175(34.4)

Verwendet man anstelle der Mischung aus Kalium- und Cäsiumfluorid das Rubidiumfluorid und arbeitet im übrigen wie im vorstehenden Beispiel angegeben, so gelangt man zu einem ähnlichen Ergebnis.

**Patentansprüche**

**1.** 2-Chlor-4,5-difluorbenzonitril der Formel

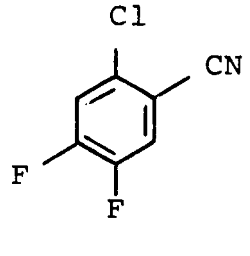

**2.** Verfahren zur Herstellung von 2-Chlor-4,5-difluorbenzonitril der Formel

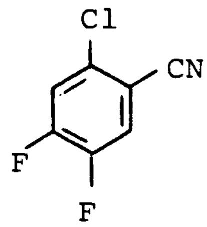

dadurch gekennzeichnet, daß man 2,4-Dichlor-5-fluorbenzonitril der Formel

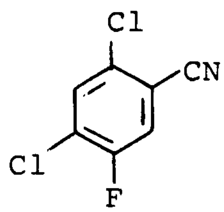

mit Kalium-, Rubidium- oder Cäsiumfluorid oder einem Gemisch daraus in einem dipolar aprotischen Lösungsmittel, gegebenenfalls in Gegenwart eines Phasentransferkatalysators, bei Temperaturen von etwa 80 bis etwa 240 ° C, umsetzt.

**3.** Verfahren nach Anspruch 2, dadurch gekennzeichnet, daß man bei Temperaturen von etwa 140 bis etwa 190 ° C umsetzt.

**4.** Verfahren nach mindestens einem der Ansprüche 2 und 3, dadurch gekennzeichnet, daß man mit einem Gemisch aus Kalium- und Cäsiumfluorid umsetz.

**5.** Verfahren nach mindestens einem der Ansprüche 2 bis 4, dadurch gekennzeichnet, daß man in Tetramethylensulfon, Dimethylsulfoxid, Tetramethylensulfoxid, Dimethylacetamid, Dimethylformamid oder N-Methylpyrrolidon als dipolar aprotischem Lösungsmittel umsetzt.

**6.** Verfahren nach mindestens einem der Ansprüche 2 bis 5, dadurch gekennzeichnet, daß man die Umsetzung in Gegenwart eines Phasentransferkatalysators durchführt.

**7.** Verfahren nach Anspruch 6, dadurch gekennzeichnet, daß der Phasentransferkatalysator ein quartäres Ammonium- oder Phosphoniumsalz ist.

**8.** Verfahren nach Anspruch 6, dadurch gekennzeichnet, daß der Phasentransferkatalysator ein Tetra-($C_1$-$C_{18}$-alkyl)-ammoniumchlorid oder -bromid, ein Tetra-($C_{1-18}$-alkyl)-phosphoniumchlorid oder -bromid, Tetraphenylphosphoniumchlorid oder -bromid oder ein [(Phenyl)$_m$ ($C_1$-$C_{18}$-alkyl)$_n$]-phosphoniumchlorid oder -bromid, wobei m = 1 bis 3, n = 3 bis 1 und m + n = 4 ist.

**9.** Verfahren nach Anspruch 6, dadurch gekennzeichnet, daß der Phasentransferkatalysator Tetramethylammoniumchlorid oder -bromid oder Octadecyltrimethyl-ammoniumchlorid oder -bromid ist.

**Patentansprüche für folgenden Vertragsstaat : ES**

**1.** Verfahren zur Herstellung von 2-Chlor-4,5-difluorbenzonitril, dadurch gekennzeichnet, daß man 2,4-Dichlor-5-fluorbenzonitril mit Kalium-, Rubidium- oder Cäsiumfluorid oder einem Gemisch daraus in einem dipolar aprotischen Lösungsmittel, gegebenenfalls in Gegenwart eines Phasentransferkatalysators, bei Temperaturen von etwa 80 bis etwa 240 ° C umsetzt.

**2.** Verfahren nach Anspruch 1, dadurch gekennzeichnet, daß man bei Temperaturen von etwa 140 bis etwa 190 ° C umsetzt.

**3.** Verfahren nach Anspruch 1 oder 2, dadurch gekennzeichnet, daß man mit einem Gemisch aus Kalium- und Cäsiumfluorid umsetzt.

**4.** Verfahren nach mindestens einem der Ansprüche 1 bis 3, dadurch gekennzeichnet, daß man in Tetramethylensulfon, Dimethylsulfoxid, Tetramethylensulfoxid, Dimethylacetamid, Dimethylformamid oder N-Methylpyrrolidon als dipolar aprotischem Lösungsmittel umsetzt.

**5.** Verfahren nach mindestens einem der Ansprüche 1 bis 4, dadurch gekennzeichnet, daß man die Umsetzung in Gegenwart eines Phasentransferkatalysators durchführt.

**6.** Verfahren nach Anspruch 5, dadurch gekennzeichnet, daß der Phasentransferkatalysator ein quartäres Ammonium- oder Phosphoniumsalz ist.

**7.** Verfahren nach Anspruch 5, dadurch gekennzeichnet, daß der Phasentransferkatalysator ein Tetra($C_1$-$C_{18}$-alkyl)-ammoniumchlorid oder -bromid, ein Tetra-($C_{1-18}$-alkyl)-phosphoniumchlorid oder -bromid, Tetraphenylphosphoniumchlorid oder -bromid oder ein [$(Phenyl)_m$ ($C_1$-$C_{18}$-$alkyl)_n$]-phosphoniumchlorid oder -bromid, wobei $m = 1$ bis 3, $n = 3$ bis 1 und $m + n = 4$ ist.

**8.** Verfahren nach Anspruch 5, dadurch gekennzeichnet, daß der Phasentransferkatalysator Tetramethylammoniumchlorid oder -bromid oder Octadecyltrimethyl-ammoniumchlorid oder -bromid ist.

Europäisches Patentamt

# EUROPÄISCHER RECHERCHENBERICHT

Nummer der Anmeldung

EP 92 10 1258

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,P | EP-A-0 433 124 (ISOCHEM)<br>* Ansprüche 6,7 *<br>--- | 1-5 | C 07 C 255/50 |
| X,P | EP-A-0 431 373 (ASAHI GLASS ET AL)<br>* Anspruch 1; Beispiel 2 *<br>--- | 1-5 | |
| A | EP-A-0 372 621 (THE DOW CHEMICAL COMPANY)<br>* Anspruch 1 *<br>----- | 2 | |
| | | | RECHERCHIERTE SACHGEBIETE (Int. Cl.5) |
| | | | C 07 C |

Der vorliegende Recherchenbericht wurde für alle Patentansprüche erstellt

| Recherchenort | Abschlußdatum der Recherche | Prüfer |
|---|---|---|
| BERLIN | 15-04-1992 | KAPTEYN H G |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)